(19) Europäisches Patentamt
European Patent Office
Office européen des brevets

(11) **EP 2 347 178 B1**

(12) **EUROPÄISCHE PATENTSCHRIFT**

(45) Veröffentlichungstag und Bekanntmachung des Hinweises auf die Patenterteilung:
**26.04.2017 Patentblatt 2017/17**

(21) Anmeldenummer: **09763896.9**

(22) Anmeldetag: **10.11.2009**

(51) Int Cl.:
*F23D 1/00* (2006.01)      *F23N 3/00* (2006.01)
*F23N 5/00* (2006.01)      *F23N 5/02* (2006.01)

(86) Internationale Anmeldenummer:
**PCT/EP2009/064872**

(87) Internationale Veröffentlichungsnummer:
**WO 2010/055020 (20.05.2010 Gazette 2010/20)**

(54) **VERFAHREN UND VORRICHTUNG ZUM ÜBERWACHEN DER VERBRENNUNG VON BRENNMATERIAL IN EINEM KRAFTWERK**

METHOD AND DEVICE FOR MONITORING THE COMBUSTION OF FUEL IN A POWER STATION

PROCÉDÉ ET DISPOSITIF POUR LA SURVEILLANCE DE LA COMBUSTION DE MATIÈRE COMBUSTIBLE DANS UNE CENTRALE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **11.11.2008 DE 102008056675**

(43) Veröffentlichungstag der Anmeldung:
**27.07.2011 Patentblatt 2011/30**

(73) Patentinhaber: **Siemens Aktiengesellschaft**
**80333 München (DE)**

(72) Erfinder:
• **MEERBECK, Bernhard**
**65779 Kelkheim (DE)**
• **SPEH, Rainer**
**64331 Weiterstadt (DE)**

(56) Entgegenhaltungen:
**WO-A1-99/39137      WO-A2-2007/062019
DE-A1- 19 714 073**

EP 2 347 178 B1

**Beschreibung**

Die Erfindung betrifft ein Verfahren und eine Vorrichtung zum Überwachen der Verbrennung von Brennmaterial in einem Verbrennungsraum eines Kraftwerks bei dem eine reale Konzentrationsverteilung und eine Temperaturverteilung eines Stoffes im Verbrennungsraum gemessen wird. Ein solches Verfahren und eine solche Vorrichtung sind zum Beispiel aus dem Dokument WO 99/39137 bekannt.

Bei Kraftwerken ist es das grundlegende Ziel, die in einem Verbrennungsraum des Kraftwerks, beispielsweise einem Kessel mit einer quadratischen Grundfläche von 10 Meter mal 10 Meter, stattfindende Verbrennung möglichst großflächig zu überwachen, um daraus die notwendigen Größen für die Optimierung des Verbrennungsprozesses ableiten zu können.

So ist als Verfahren die Absorptionsspektroskopie bekannt. Als alternative Messtechnik ist die Schall-Pyrometrie bekannt. Mit Absorptionsspektroskopie oder Schall-Pyrometrie können nur Mittelwerte einer Linie im Kesselraum bzw. Verbrennungsraum gemessen werden.

Zum Berechnen der Temperatur- und Konzentrationsverteilung in einer Ebene eines Verbrennungsraums aus gemessenen Mittelwerten an verschiedenen Stellen des Verbrennungsraumes eines Kraftwerks ist die CAT-Messtechnik, die Computer Aided Tomographie, bekannt.

Es ist eine Aufgabe der Erfindung eine weitergehende Überwachung der Verbrennung in einem Kraftwerk zu ermöglichen, um damit die Grundlage für die Optimierung des Verbrennungsprozesses zu liefern.

Die Aufgabe ist erfindungsgemäß mit einem Verfahren gemäß Anspruch 1 und einer Vorrichtung gemäß Anspruch 6 gelöst. Vorteilhafte Weiterbildungen sind in den abhängigen Ansprüchen beschrieben.

Das erfindungsgemäße Verfahren zum Überwachen der Verbrennung von Brennmaterial in einem Verbrennungsraum eines Kraftwerks umfasst die Schritte:

- Messen einer realen Konzentrationsverteilung und einer Temperaturverteilung eines Stoffes in zwei Messebenen des Verbrennungsraums, wobei die Messung auf einer Kombination aus Messtechnik und Computer Aided Tomography (CAT)-Technik beruht, und am Rand der zwei Messebenen sich jeweils beabstandet voneinander Messinstrumente befinden, die eine linienförmige Messung in den zugehörigen Messebenen ermöglichen,
- Auswerten der realen Konzentrationsverteilung, wobei beim Auswerten ein Mittelwert in mindestens einer Dimension einer gemessenen zweidimensionalen Konzentrationsverteilung gebildet wird und dabei die Verbrennungsstöchiometrie berücksichtigt wird und
- Rückschließen auf die quantitative Zusammensetzung des Brennmaterials auf der Grundlage der vorgenommenen Auswertung.

Die erfindungsgemäße Vorrichtung zum Überwachen der Verbrennung von Brennmaterial in einem Verbrennungsraum eines Kraftwerks umfasst entsprechende Einrichtungen, welche zur Durchführung der Verfahrensschritte ausgebildet sind.

Mit anderen Worten liegt der Grundgedanke der Erfindung darin, dass mittels einer Konzentrationsmessung mindestens eines Stoffes, wie beispielsweise einem Gasbestandteil des Abgases der Verbrennung, eine quantitative Eigenschaft von Brennmaterial festgestellt werden kann. Es kann mit der Berücksichtigung der Verbrennungsstöchiometrie festgestellt werden, ob ein bestimmter Bestandteil in dem untersuchten Stoff in jener Quantität vorkommt, wie sich aufgrund der Verbrennungsstöchiometrie ergeben müsste.

So kann insbesondere vorteilhaft auf der Grundlage mehrerer gemessener Konzentrationswerte für bevorzugt die Stoffe $CO_2$ (Kohlendioxid), CO (Kohlenmonoxid), $O_2$.(Sauerstoff), $H_2O$ (Wasser) und/oder $N_2$ (Stickstoff) eine quantitative Bestimmung der Zusammensetzung von Brennmaterial, wie insbesondere

Kohle, erfolgen. Bevorzugt wird dabei die Verbrennungsstöchiometrie wie folgt verwendet:

$$CH_aS_bO_cN_d + fH_2O + B(1 + E)(O_2 + 3,76N_2 + wH_2O)$$

$$= (1 - x - m)CO_2 + xC + mCO + [\frac{a}{2} + f + B(1 + E)w]H_2O + bSO_2$$

$$+ [BE + x + \frac{m}{2}]O_2 + [3,76B(1 + E) + \frac{d}{2}]N_2$$

wobei ein Luftüberschuss von $\lambda = 1 + E$ angenommen wird und die Parameter a, b und c anhand der gemessenen Konzentrationen ermittelt werden, während bevorzugt zur Vereinfachung d = 0 angenommen wird.

Bei der erfindungsgemäßen Lösung wird beim Auswerten ein Mittelwert in mindestens einer Dimension einer gemessenen zweidimensionalen Konzentrationsverteilung gebildet.

Bei einer vorteilhaften Weiterbildung der erfindungsgemäßen Lösung wird beim Messen in zwei Ebenen des Verbrennungsraums je eine Konzentrationsverteilung ermittelt und zwischen diesen Messungen erfolgt zur Erhöhung der Messsicherheit eine Plausibilitätskontrolle.

Bei einer vorteilhaften Weiterbildung der erfindungsgemäßen Lösung erfolgt zum Schaffen eines rechnertechnisch besonders einfachen Vorgehens beim Rückschließen auf die Zusammensetzung von verbranntem Brennmaterial ein Vergleich zwischen einer mit einem Musterbrennmaterial ermittelten Konzentrationsverteilung und dessen Verbrennungsstöchiometrie und der gemessenen realen Konzentrationsverteilung.

Bei einer vorteilhaften Weiterbildung der erfindungsgemäßen Lösung erfolgt beim Rückschließen auf die Zusammensetzung von verbranntem Brennmaterial ein Vergleich mit mindestens einer gespeicherten charakteristischen Konzentrationsverteilung eines Musterbrennmaterials. Es ist auf diese Art und Weise ein rechnertechnisch besonders schnelles Vorgehen möglich.

Bei einer vorteilhaften Weiterbildung der erfindungsgemäßen Lösung erfolgt das Rückschließen auf die Zusammensetzung des Brennmaterials zeitgleich mit dem Messen. Insgesamt kann so auf einfache Weise eine zwar nur genäherte, dafür aber sehr zeitnahe Erkennung der quantitativen Zusammensetzung von Brennmaterial auf kostengünstige, einfache und zugleich prozesssichere Art erzielt werden.

Bei der obigen Definition der Erfindung wird unter "Stoff" allgemein jede Art von Verbrennungsprodukt verstanden, insbesondere in Form von Gas als Bestandteil des Abgases. Ferner wird unter dem Begriff Brennmaterial jede Art von Material verstanden, welches bei Kraftwerken zur Verbrennung gelangt. Für die vorliegend besonders relevanten Kohlekraftwerke sind dies unterschiedliche Kohlen bzw. unterschiedliche Kohlesorten.

Die genannten vorteilhaften Weiterbildungen des erfindungsgemäßen Verfahrens sind bevorzugt in Gestalt entsprechend angepasster Einrichtungen auch in den erfindungsgemäßen Vorrichtungen realisiert.

Nachfolgend wird ein Ausführungsbeispiel der erfindungsgemäßen Lösung anhand der beigefügten schematischen Zeichnungen näher erläutert.

Es zeigen:

Fig. 1    ein Ausführungsbeispiel der erfindungsgemäßen Vorrichtung und

Fig. 2    ein Ausführungsbeispiel des erfindungsgemäßen Verfahrens.

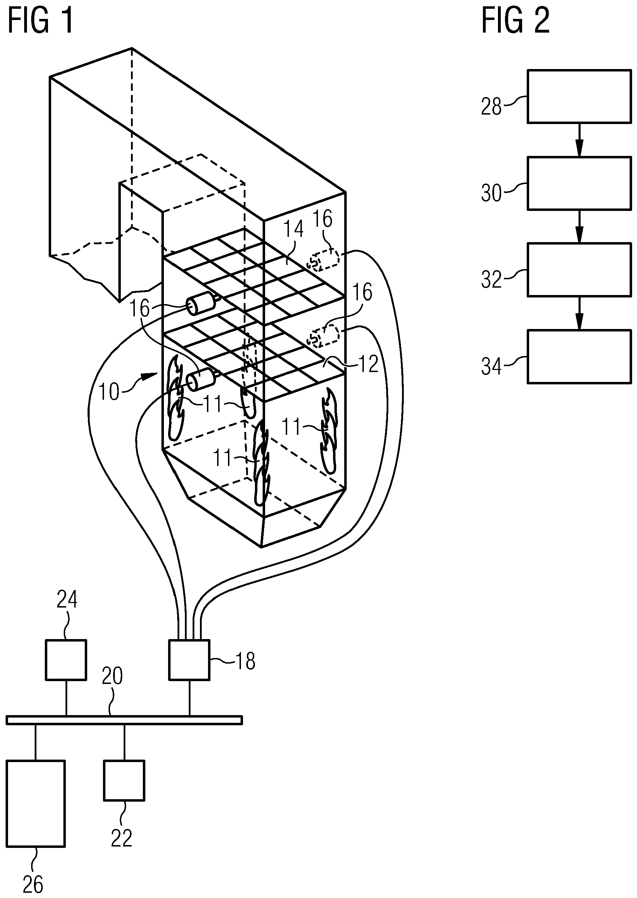

In Fig. 1 ist ein Verbrennungsraum 10 eines weiter nicht veranschaulichten Kohlekraftwerks dargestellt, in dem beim Betrieb des Kohlekraftwerks ein Kohlefeuer brennt. In dem Verbrennungsraum 10 befinden sich dabei das Brennmaterial Kohle mit zugehörigen Brenngasen, mehrere Flammen 11 sowie Abgase.

Im Verbrennungsraum 10 sind zwei Messebenen 12 und 14 vorgesehen, an deren Rand sich jeweils beabstandet von einander Messinstrumente 16 befinden. Jeweils zwei der Messinstrumente 16 ermöglichen eine linienförmige Messung in der zugehörigen Messebene 12 bzw. 14, wobei mit Hilfe der Messinstrumente 16 und einer zugehörigen Auswerteeinrichtung 18 z. B. die Konzentration der Stoffe $O_2$ (Sauerstoff) und CO (Kohlenmonoxid) gemessen werden können.

Ferner kann mit den Messinstrumenten 16 und der Auswerteeinrichtung 18 die Temperaturverteilung in der zugehörigen Messebene 12 bzw. 14 ermittelt werden. Die Messung beruht dabei auf einer Kombination von Messtechnik und CAT-Berechnung.

Die Auswerteeinrichtung 18 ist über einen Datenbus 20 mit einer Optimierungseinrichtung 22, einer Bedieneinrichtung 24 und einer Leiteinrichtung bzw. Leittechnik 26 betrieblich gekoppelt. Über die Bedieneinrichtung 24 können die von der Auswerteeinrichtung 18 gemessenen realen Konzentrationsverteilungen sowie Temperaturverteilungen in den Ebenen 12 und 14 derart genutzt werden, dass die Optimierungseinrichtung 22 daraus auf die quantitative Zusammensetzung des im Verbrennungsraum 10 aktuell verbrannten Brennmaterials, vorliegend auf die dortige quantitative Kohlezusammensetzung, schließen kann.

Die quantitative Zusammensetzung des Brennmaterials wird ermittelt, z. B. um die in dem Verbrennungsraum 10 brennenden Flammen 11 insbesondere im Hinblick auf einen geringen Ausstoß von $NO_x$ (Stickoxid) zu optimieren.

Zur Ermittlung der quantitativen Zusammensetzung des Brennmaterials verwendet bzw. berücksichtigt die Optimierungseinrichtung 22 die allgemeine Verbrennungsstöchiometrie, wie sie in der oben genannten Formel wiedergegeben ist. Die real gemessenen Verteilungen von Konzentration und Temperatur werden dazu zunächst längs einer Dimension insbesondere in einer Ebene gemittelt und gegenseitig auf ihre Plausibilität geprüft.

Das zugehörige Verfahren ist in Fig. 2 veranschaulicht. Es umfasst den Schritt 28 des Messens der Konzentrationsverteilung der Stoffe $N_2$, $SO_2$, $CO_2$, $O_2$ und CO und der Temperaturverteilung in der Ebene 12. Im Schritt 30 wird zeitgleich die Konzentrationsverteilung der Stoffe $N_2$, $SO_2$, $CO_2$, $O_2$ und CO in der Ebene 14 sowie die dortige Temperaturverteilung gemessen.

Nicht gemessene Konzentrationen können dabei durch die Gleichungen der Verbrennungsstöchiometrie oder Messungen an anderer Stelle ermittelt werden.

In den Schritten 28 und 30 erfolgt dabei auch eine Plausibilitätskontrolle der in den beiden Ebenen 12 und 14 des Verbrennungsraums 10 ermittelten Konzentrationsverteilungen sowie eine Mittelwertbildung über die jeweils zweidimensional gemessenen Konzentrationen.

Im Schritt 32 wird, wie oben erläutert, die Konzentrationsverteilung sowie die Temperaturverteilung in den Ebenen 12 und 14 unter Berücksichtigung der Verbrennungsstöchiometrie derart ausgewertet, dass in einem Schritt 34 auf die quantitative Zusammensetzung des Brennmaterials im Verbrennungsraum 10 rückgeschlossen werden kann.

Auf der Grundlage dieses Rückschlusses erfolgt dann in einem Schritt 34 eine Optimierung der Verbrennung, beispielsweise durch eine Änderung der Luftschichtung und/oder ein abschnittsweises Ändern des Luftüberschusses.

## Patentansprüche

1.  Verfahren zum Überwachen der Verbrennung von Brennmaterial in einem Verbrennungsraum (10) eines Kraftwerks mit den Schritten:

    - Messen (28, 30) einer realen Konzentrationsverteilung und einer Temperaturverteilung eines Stoffes in zwei Messebenen (12, 14) des Verbrennungsraums (10), wobei die Messung auf einer Kombination aus Messtechnik und Computer Aided Tomography (CAT)-Technik beruht, und am Rand der zwei Messebenen (12, 14) sich jeweils beabstandet voneinander Messinstrumente (16) befinden, die eine linienförmige Messung in den zugehörigen Messebenen (12, 14) ermöglichen,
    - Auswerten (32) der realen Konzentrationsverteilung und der realen Temperaturverteilung, wobei beim Auswerten (32) ein Mittelwert in mindestens einer Dimension einer gemessenen zweidimensionalen Konzentrationsverteilung gebildet wird und dabei die Verbrennungsstöchiometrie berücksichtigt wird und
    - Rückschließen (34) auf die quantitative Zusammensetzung des Brennmaterials auf der Grundlage der vorgenommenen Auswertung (32).

2.  Verfahren nach Anspruch 1,
    bei dem beim Messen (28, 30) in zwei Messebenen (12, 14) des Verbrennungsraums je eine Konzentrations- und Temperaturverteilung ermittelt wird und zwischen diesen Messungen eine Plausibilitätskontrolle erfolgt.

3.  Verfahren nach Anspruch 1 oder 2,
    bei dem beim Rückschließen (34) auf die quantitative Zusammensetzung des Brennmaterials ein Vergleich zwischen einer mit einem Musterbrennmaterial ermittelten Konzentrationsverteilung und dessen Verbrennungsstöchiometrie und der gemessenen realen Konzentrationsverteilung erfolgt.

4.  Verfahren nach Anspruch 3,
    bei dem beim Rückschließen (34) auf die quantitative Zusammensetzung des Brennmaterials ein Vergleich mit mindestens einer gespeicherten charakteristischen Konzentrationsverteilung eines Musterbrennmaterials erfolgt.

5.  Verfahren nach einem der Ansprüche 1 bis 4,
    bei dem das Rückschließen (34) auf die quantitative Zusammensetzung des Brennmaterials zeitgleich mit dem Messen (28, 30) erfolgt.

6.  Vorrichtung zum Überwachen der Verbrennung von Brennmaterial in einem Verbrennungsraum (10) eines Kraftwerks mit

    - einer Einrichtung (16), welche zum Messen einer realen Konzentrationsverteilung und einer realen Temperaturverteilung eines Stoffes in zwei Messebenen (12, 14) des Verbrennungsraums (10) ausgebildet ist, wobei die Messung auf einer Kombination aus Messtechnik und Computer Aided Tomography (CAT)-Technik beruht, und am Rand der zwei Messebenen (12, 14) sich jeweils beabstandet voneinander Messinstrumente (16) befinden, die eine linienförmige Messung in den zugehörigen Messebenen (12, 14) ermöglichen,
    - einer Einrichtung, welche zum Auswerten der realen Konzentrationsverteilung und Temperaturverteilung mit Berücksichtigung der Verbrennungsstöchiometrie ausgebildet ist, wobei beim Auswerten (32) ein Mittelwert in mindestens einer Dimension einer gemessenen zweidimensionalen Konzentrationsverteilung gebildet wird und dabei die Verbrennungsstöchiometrie berücksichtigt wird und
    - einer Einrichtung (22), welche zum Rückschließen auf die quantitative Zusammensetzung des Brennmaterials auf der Grundlage der vorgenommenen Auswertung ausgebildet ist.

**Claims**

1. Method for monitoring the combustion of fuel in a combustion chamber (10) in a power station, having the steps:

   - measure (28, 30) an actual concentration distribution and a temperature distribution of a substance in two measurement planes (12, 14) of the combustion chamber (10), wherein the measurement is based on a combination of measurement technology and Computer Aided Tomography (CAT) technology, and on the edge of the two measurement planes (12, 14) there are in each case measuring instruments (16), spaced apart from each other, which permit a measurement along a line in the associated measurement planes (12, 14),
   - analyse (32) the actual concentration distribution and the actual temperature distribution, wherein, when making the analysis (32), a mean value is formed in at least one dimension of a measured two-dimensional concentration distribution and this takes into account the combustion stochiometry, and
   - draw conclusions (34) about the quantitative composition of the fuel on the basis of the analysis (32) undertaken.

2. Method according to claim 1,
   wherein, when making the measurements (28, 30) in two measurement planes (12, 14) in the combustion chamber, in each case a concentration and temperature distribution is determined, and a plausibility check between these measurements is carried out.

3. Method according to claim 1 or 2,
   wherein, when drawing conclusions (34) about the quantitative composition of the fuel, a comparison is made between a concentration distribution and its combustion stochiometry determined with a fuel sample and the measured actual concentration distribution.

4. Method according to claim 3,
   wherein, when drawing conclusions (34) about the quantitative composition of the fuel, a comparison is made with at least one stored characteristic concentration distribution for a fuel sample.

5. Method according to one of claims 1 to 4,
   wherein the drawing of conclusions (34) about the quantitative composition of the fuel is effected at the same time as the measurements are made (28, 30).

6. Device for monitoring the combustion of fuel in a combustion chamber (10) in a power station, with

   - equipment (16) which is embodied for measuring an actual concentration distribution and an actual temperature distribution of a substance in two measurement planes (12, 14) of the combustion chamber (10), wherein the measurement is based on a combination of measurement technology and Computer Aided Tomography (CAT) technology, and on the edge of the two measurement planes (12, 14) there are in each case measuring instruments (16), spaced apart from each other, which permit a measurement along a line in the associated measurement planes (12, 14),
   - equipment which is embodied for analysing the actual concentration distribution and actual temperature distribution, taking into account the combustion stochiometry, wherein, when making the analysis (32), a mean value is formed in at least one dimension of a measured two-dimensional concentration distribution and this takes into account the combustion stochiometry and
   - equipment (22) which is embodied for drawing conclusions about the quantitative composition of the fuel on the basis of the analysis undertaken.

**Revendications**

1. Procédé de contrôle de la combustion de matière combustible dans un espace ( 10 ) de combustion d'une centrale électrique, comprenant les stades :

   - Mesure ( 28, 30 ) d'une répartition réelle de concentration et d'une répartition réelle de température d'une substance dans deux plans ( 12, 14 ) de mesure de l'espace ( 10 ) de combustion, la mesure reposant sur une combinaison d'une technique de mesure et d'une technique de tomographie assistée par ordinateur ( CAT ) et il se trouve, au bord des deux plans ( 12, 14 ) de mesure, des instruments ( 16 ) de mesure à distance l'un de l'autre qui rendent possible une mesure linéaire dans les plans ( 12, 14 ) de mesure associés,

- Exploitation ( 32 ) de la répartition réelle de concentration et de la répartition réelle de température,

dans lequel,
lors de l'exploitation ( 32 ), on forme une valeur moyenne dans au moins une dimension d'une répartition de concentration mesurée en deux dimensions et on tient compte à cet effet de la stoechiométrie de combustion et

- Déduction ( 34 ) de la composition quantitative de la matière de combustible sur la base de l'exploitation ( 32 ) effectuée.

2. Procédé suivant la revendication 1,
dans lequel, lors de la mesure ( 28, 30 ) dans deux plans ( 12, 14 ) de mesure de l'espace de combustion, on détermine respectivement une répartition de concentration et de température et on effectue un contrôle de vraisemblance entre ces mesures.

3. Procédé suivant la revendication 1 ou 2,
dans lequel, lors de la déduction ( 34 ) de la composition quantitative de la matière combustible, on effectue une comparaison entre une répartition de concentration déterminée avec une matière combustible modèle et sa stoechiométrie de combustion et la répartition réelle de concentration mesurée.

4. Procédé suivant la revendication 3,
dans lequel, lors de la déduction ( 34 ) de la composition quantitative de la matière combustible, on effectue une comparaison à au moins une répartition de concentration caractéristique mémorisée d'une matière combustible modèle.

5. Procédé suivant l'une des revendications 1 à 4,
dans lequel on effectue la déduction ( 34 ) de la composition quantitative de la matière combustible en même temps que les mesures ( 28, 30 ).

6. Système de contrôle de la combustion de matière combustible dans un espace ( 10 ) de combustion d'une centrale électrique comprenant :

- un dispositif ( 16 ) constitué pour mesurer une répartition réelle de concentration et une répartition réelle de température d'une substance dans deux plans ( 12, 14 ) de mesure de l'espace ( 10 ) de combustion, la mesure reposant sur une combinaison d'une technique de mesure et d'une technique de tomographie assistée par ordinateur ( CAT ) et, au bord des deux plans ( 12, 14 ) de mesure, se trouvent respectivement des instruments ( 16 ) de mesure à distance l'un de l'autre qui rendent possible une mesure linéaire dans les plans ( 12, 14 ) de mesure associés,
- un dispositif constitué pour exploiter la répartition réelle de concentration et la répartition réelle de température en tenant compte de la stoechiométrie de la combustion, dans lequel, lors de l'exploitation ( 32 ), on forme une valeur moyenne dans au moins une dimension d'une répartition de concentration à deux dimensions mesurée et on tient compte à cet effet de la stoechiométrie de la combustion et
- un dispositif ( 22 ) constitué pour déduire la composition quantitative de la matière combustible sur la base de l'exploitation effectuée.

FIG 1

FIG 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9939137 A